# EUROPEAN PATENT SPECIFICATION

(11) **EP 0 692 026 B1**
(45) Date of publication and mention of the grant of the patent: **17.03.1999**
(21) Application number: 94906667.4
(22) Date of filing: 28.01.1994
(51) Int. Cl.: C12N 15/12, C07K 14/725, C12N 5/10, C12N 1/21, C12P 21/08, C12Q 1/68, G01N 33/68, A01K 67/027, C07H 21/00

(54) **A HUMAN T-CELL RECEPTOR OF THE G-PROTEIN COUPLED RECEPTOR FAMILY**
EIN MENSCHLICHER T-ZELL REZEPTOR DER PROTEIN G-GEKOPPELTE REZEPTOR FAMILIE
RECEPTEUR DE LYMPHOCYTE T HUMAIN APPARTENANT A LA FAMILLE DES RECEPTEURS COUPLES A UNE PROTEINE G

(30) Priority: 09.04.1993 US 46531
(43) Date of publication of application: 17.01.1996
(73) Proprietor: PFIZER INC., New York, N.Y. 10017 (US)
(72) Inventor: HANKE, Jeffrey Herbert, Gales Ferry, CT 06335 (US); OGBORNE, Kevin Thomas, Clinton, CT 06413 (US)
(74) Representative: Hayles, James Richard
(86) International application number: US9400598
(87) International publication number: WO9424282

(56) References cited:
- CURRENT OPINION IN STRUCTURAL BIOLOGY vol. 1, no. 3 , June 1991 pages 394 - 401 KERLAVAGE, A.R.; 'G-protein coupled receptor family'
- TRENDS IN PHARMACOLOGICAL SCIENCES vol. 11 , December 1990 , CAMBRIDGE GB pages 492 - 499 FINDLAY, J. ET AL.; 'Three-dimensional modelling of G protein-linked receptors.'
- SCIENCE vol. 240 , 10 June 1988 , LANCASTER, PA pages 1468 - 1474 JAENISCH, R.; 'Transgenic animals'
- SCIENCE vol. 261 , 20 August 1993 , LANCASTER, PA pages 1004 - 1012 STEIN, C.A. ET AL.; 'Antisense oligonucleotides as therapeutic agents - Is the bullet really magical ?'

### SEQUENCE LISTING

### (1) GENERAL INFORMATION:

(i) APPLICANT:
   Hanke, Jeffrey H.
   Ogborne, Kevin T.
   Pfizer Inc., (non-U.S.)
(ii) TITLE OF INVENTION: HUMAN RECEPTOR
(iii) NUMBER OF SEQUENCES: 10
(iv) CORRESPONDENCE ADDRESS:
   (A) ADDRESSEE: Gregg C. Benson, Pfizer Inc
   (B) STREET: Eastern Point Road
   (C) CITY: Groton
   (D) STATE: Connecticut
   (E) COUNTRY: U.S.A.
   (F) ZIP: 06340
(v) COMPUTER READABLE FORM:
   (A) MEDIUM TYPE: Floppy disk
   (B) COMPUTER: IBM PC compatible
   (C) OPERATING SYSTEM: PC-DOS/MS-DOS
   (D) SOFTWARE: PatentIn Release #1.0, Version #1.25
(vi) CURRENT APPLICATION DATA:
   (A) APPLICATION NUMBER:
   (B) FILING DATE:
   (C) CLASSIFICATION:
(viii) ATTORNEY/AGENT INFORMATION:
   (A) NAME: Benson, Gregg C.
   (B) REGISTRATION NUMBER: 30,997
   (C) REFERENCE/DOCKET NUMBER: PC8348AGCB
(ix) TELECOMMUNICATION INFORMATION:
   (A) TELEPHONE: (203) 441-4901
   (B) TELEFAX: (203) 441-5221

### (2) INFORMATION FOR SEQ ID NO:1:

(i) SEQUENCE CHARACTERISTICS:
   (A) LENGTH: 1613 base pairs
   (B) TYPE: nucleic acid
   (C) STRANDEDNESS: double
   (D) TOPOLOGY: linear
(ii) MOLECULE TYPE: cDNA
(xi) SEQUENCE DESCRIPTION: SEQ ID NO:1:
   CGGATTGTGA ATTTAGTTTT GAACCATCAT TAGGCAAAGA TAGCTTCTCT AGAGAGAATC    60
   ATGCCTGCTA ATTACACGTG TACCAGGCCA GATGGAGACA ATACAGATTT TCGATACTTT   120
   ATTTATGCAG TGACATACAC TGTCATTCTT GTGCCAGGTC TCATAGGGAA TATATTAGCC   180
   CTGTGGGTAT TCTATGGTTA TATGAAAGAA ACCAAACGAG CTGTGATATT TATGATAAAC   240
   TTAGCCATTG CTGACTTACT ACAAGTTCTT TCCTTGCCAC TGAGGATCTT CTACTACTTG   300
   AATCATGACT GGCCATTTGG GCCTGGTCTC TGCATGTTCT GTTTCTACCT GAAGTATGTC   360
   AACATGTATG CAAGCATCTA CTTCTTGGTC TGCATCAGTG TGCGACGATT TTGGTTTCTC   420
   ATGTACCCCT TTCGCTTCCA TGACTGCAAA CAGAAATATG ACCTGTACAT CAGCATTGCT   480
   GGCTGGCTGA TCATCTGCCT TGCCTGTGTA CTCTTTCCAC TCCTCAGAAC CAGTGATGAT   540
   ACCCCTGGCA ATAGGACCAA ATGCTTTGTG GATCTTCCTA CCAGGAATGT CAACCTGGCC   600
   CAGTCCGTTG TTATGATGAC CATTGGCGAG TTGATTGGGT TTGTAACTCC GCTTCTGATT   660
   GTCCTATATT GTACCTGGAA GACGGTTTTA TCACTGCAAG ATAAATATCC CATGGCCCAA   720
   GATCTTGGAG AGAAACAGAA AGCCTTGAAG ATGATTCTAA CCTGTGCAGG GGTATTCCTA   780
   ATTTGCTTTG CACCTTATCA TTTCAGTTTT CCTTTAGATT TCCTGGTGAA GTCCAATGAA   840
   ATTAAAAGCT GCCTAGCCAG AAGGGTGATT CTAATATTTC ATTCTGTGGC ATTGTGTCTT   900
   GCTAGTCTGA ATTCATGTCT TGACCCAGTC ATATACTACT TTTCCACTAA TGAGTTCCGA   960
   AGACGGCTTT CAAGACAAGA TTTGCATGAC AGCATCCAAC TCCATGCAAA ATCCTTTGTG  1020
   AGTAACCATA CAGCTTCCAC CATGACACCT GAATTATGCT AAAACAAAAA ACCAAACTGA  1080
   ATGTGACCTG AAATGCAAGT ACATCAGAAC ATATCTGCAA TACCCAAGCC ACAGGGAAGA  1140
   ACTTGCAAAA CAACACAGCT TTTCAGTTCT GCTCTATCTT ACTGCTATGG GGAATTCACT  1200
   TCTTCAAAGC AGGACCTATT TGGAGCATTA CGATCCACGA TTATTGATGT TGACATGTCC  1260
   ATGTAGTAAT TTTTCTTCAA GTCTGTAAAT CTTAAAATAT CAAATTTCTG TGACATCCTA  1320
   TAAACATATG CACTCAACTG TAGTCAGTAC TTTTACCTGT GAACCTCAGG CACAAAAAGA  1380
   TTATTAGCTT GGAGTCACCA TAAACATTTA GTTTTGTTGC AACAGATACT GAGTCTTTAT  1440
   GTTCAGAGGA AATGTAAGTG TCTTTTTATA TTAGTAATCA CAGTTTACAT GCCATTTTCA  1500
   TATGTTTGGT TATATTTTAG TGGGATAGAT GATATATTAC CCTGTGTAAA GAAAATGTAA  1560
   ACATAAGATC ATTTTTATCT CTCAAGTGTG ATTACTCTTC AGAGTTTGAA CCG         1613

### (2) INFORMATION FOR SEQ ID NO:2:

(i) SEQUENCE CHARACTERISTICS:
   (A) LENGTH: 333 amino acids
   (B) TYPE: amino acid
   (D) TOPOLOGY: linear
(ii) MOLECULE TYPE: protein
(xi) SEQUENCE DESCRIPTION: SEQ ID NO:2:
   Met Pro Ala Asn Tyr Thr Cys Thr Arg Pro Asp Gly Asp Asn Thr Asp
   1               5                   10                  15
   Phe Arg Tyr Phe Ile Tyr Ala Val Thr Tyr Thr Val Ile Leu Val Pro
               20                  25                  30
   Gly Leu Ile Gly Asn Ile Leu Ala Leu Trp Val Phe Tyr Gly Tyr Met
           35                  40                  45
   Lys Glu Thr Lys Arg Ala Val Ile Phe Met Ile Asn Leu Ala Ile Ala
       50                  55                  60
   Asp Leu Leu Gln Val Leu Ser Leu Pro Leu Arg Ile Phe Tyr Tyr Leu
   65                  70                  75                  80
   Asn His Asp Trp Pro Phe Gly Pro Gly Leu Cys Met Phe Cys Phe Tyr
                   85                  90                  95
   Leu Lys Tyr Val Asn Met Tyr Ala Ser Ile Tyr Phe Leu Val Cys Ile
               100                 105                 110
   Ser Val Arg Arg Phe Trp Phe Leu Met Tyr Pro Phe Arg Phe His Asp
           115                 120                 125
   Cys Lys Gln Lys Tyr Asp Leu Tyr Ile Ser Ile Ala Gly Trp Leu Ile
       130                 135                 140
   Ile Cys Leu Ala Cys Val Leu Phe Pro Leu Leu Arg Thr Ser Asp Asp
   145                 150                 155                 160
   Thr Pro Gly Asn Arg Thr Lys Cys Phe Val Asp Leu Pro Thr Arg Asn
                   165                 170                 175
   Val Asn Leu Ala Gln Ser Val Val Met Met Thr Ile Gly Glu Leu Ile
               180                 185                 190
   Gly Phe Val Thr Pro Leu Leu Ile Val Leu Tyr Cys Thr Trp Lys Thr
           195                 200                 205
   Val Leu Ser Leu Gln Asp Lys Tyr Pro Met Ala Gln Asp Leu Gly Glu
       210                 215                 220
   Lys Gln Lys Ala Leu Lys Met Ile Leu Thr Cys Ala Gly Val Phe Leu
   225                 230                 235                 240
   Ile Cys Phe Ala Pro Tyr His Phe Ser Phe Pro Leu Asp Phe Leu Val
                   245                 250                 255
   Lys Ser Asn Glu Ile Lys Ser Cys Leu Ala Arg Arg Val Ile Leu Ile
               260                 265                 270
   Phe His Ser Val Ala Leu Cys Leu Ala Ser Leu Asn Ser Cys Leu Asp
           275                 280                 285
   Pro Val Ile Tyr Tyr Phe Ser Thr Asn Glu Phe Arg Arg Arg Leu Ser
       290                 295                 300
   Arg Gln Asp Leu His Asp Ser Ile Gln Leu His Ala Lys Ser Phe Val
   305                 310                 315                 320
   Ser Asn His Thr Ala Ser Thr Met Thr Pro Glu Leu Cys
                   325                 330

### (2) INFORMATION FOR SEQ ID NO:3:

(i) SEQUENCE CHARACTERISTICS:
   (A) LENGTH: 26 base pairs
   (B) TYPE: nucleic acid
   (C) STRANDEDNESS: single
   (D) TOPOLOGY: linear
(ii) MOLECULE TYPE: cDNA
(ix) FEATURE:
   (A) NAME/KEY: modified_base
   (B) LOCATION: 11
   (D) OTHER INFORMATION: /mod_base= OTHER /note= "c or t"
(ix) FEATURE:
   (A) NAME/KEY: modified_base
   (B) LOCATION: 12
   (D) OTHER INFORMATION: /mod_base= OTHER /note= "c or t"
(ix) FEATURE:
   (A) NAME/KEY: modified_base
   (B) LOCATION: 14
   (D) OTHER INFORMATION: /mod_base= OTHER /note= "g or a"
(ix) FEATURE:
   (A) NAME/KEY: modified_base
   (B) LOCATION: 17
   (D) OTHER INFORMATION: /mod_base= OTHER /note= "g or c"
(ix) FEATURE:
   (A) NAME/KEY: modified_base
   (B) LOCATION: 18
   (D) OTHER INFORMATION: /mod_base= OTHER /note= "g, c or t"
(ix) FEATURE:
   (A) NAME/KEY: modified_base
   (B) LOCATION: 20
   (D) OTHER INFORMATION: /mod_base= OTHER /note= "g or a"
(ix) FEATURE:
   (A) NAME/KEY: modified_base
   (B) LOCATION: 23
   (D) OTHER INFORMATION: /mod_base= OTHER /note= "c or t"
(xi) SEQUENCE DESCRIPTION: SEQ ID NO:3:
   ATAAGCTTAA NNTNGCNNTN GCNGAC 26

### (2) INFORMATION FOR SEQ ID NO:4:

(i) SEQUENCE CHARACTERISTICS:
   (A) LENGTH: 25 base pairs
   (B) TYPE: nucleic acid
   (C) STRANDEDNESS: single
   (D) TOPOLOGY: linear
(ii) MOLECULE TYPE: cDNA
(ix) FEATURE:
   (A) NAME/KEY: modified_base
   (B) LOCATION: 9
   (D) OTHER INFORMATION: /mod_base= OTHER /note= "g or c"
(ix) FEATURE:
   (A) NAME/KEY: modified_base
   (B) LOCATION: 11
   (D) OTHER INFORMATION: /mod_base= OTHER /note= "g or c"
(ix) FEATURE:
   (A) NAME/KEY: modified_base
   (B) LOCATION: 12
   (D) OTHER INFORMATION: /mod_base= OTHER /note= "g or c"
(ix) FEATURE:
   (A) NAME/KEY: modified_base
   (B) LOCATION: 14
   (D) OTHER INFORMATION: /mod_base= OTHER /note= "g or t"
(ix) FEATURE:
   (A) NAME/KEY: modified_base
   (B) LOCATION: 15
   (D) OTHER INFORMATION: /mod_base= OTHER /note= "a or g"
(ix) FEATURE:
   (A) NAME/KEY: modified_base
   (B) LOCATION: 18
   (D) OTHER INFORMATION: /mod_base= OTHER /note= "g or t"
(ix) FEATURE:
   (A) NAME/KEY: modified_base
   (B) LOCATION: 21
   (D) OTHER INFORMATION: /mod_base= OTHER /note= "g or c"
(ix) FEATURE:
   (A) NAME/KEY: modified_base
   (B) LOCATION: 22
   (D) OTHER INFORMATION: /mod_base= OTHER /note= "a or g"
(xi) SEQUENCE DESCRIPTION: SEQ ID NO:4:
   TCGAATTCNA NNTNNTANGG NNCAA 25

### (2) INFORMATION FOR SEQ ID NO:5:

(i) SEQUENCE CHARACTERISTICS:
   (A) LENGTH: 999 base pairs
   (B) TYPE: nucleic acid
   (C) STRANDEDNESS: double
   (D) TOPOLOGY: linear
(ii) MOLECULE TYPE: cDNA
(xi) SEQUENCE DESCRIPTION: SEQ ID NO:5:
   ATGCCTGCTA ATTACACGTG TACCAGGCCA GATGGAGACA ATACAGATTT TCGATACTTT     60
   ATTTATGCAG TGACATACAC TGTCATTCTT GTGCCAGGTC TCATAGGGAA TATATTAGCC    120
   CTGTGGGTAT TCTATGGTTA TATGAAAGAA ACCAAACGAG CTGTGATATT TATGATAAAC    180
   TTAGCCATTG CTGACTTACT ACAAGTTCTT TCCTTGCCAC TGAGGATCTT CTACTACTTG    240
   AATCATGACT GGCCATTTGG GCCTGGTCTC TGCATGTTCT GTTTCTACCT GAAGTATGTC    300
   AACATGTATG CAAGCATCTA CTTCTTGGTC TGCATCAGTG TGCGACGATT TTGGTTTCTC    360
   ATGTACCCCT TTCGCTTCCA TGACTGCAAA CAGAAATATG ACCTGTACAT CAGCATTGCT    420
   GGCTGGCTGA TCATCTGCCT TGCCTGTGTA CTCTTTCCAC TCCTCAGAAC CAGTGATGAT    480
   ACCCCTGGCA ATAGGACCAA ATGCTTTGTG GATCTTCCTA CCAGGAATGT CAACCTGGCC    540
   CAGTCCGTTG TTATGATGAC CATTGGCGAG TTGATTGGGT TTGTAACTCC GCTTCTGATT    600
   GTCCTATATT GTACCTGGAA GACGGTTTTA TCACTGCAAG ATAAATATCC CATGGCCCAA    660
   GATCTTGGAG AGAAACAGAA AGCCTTGAAG ATGATTCTAA CCTGTGCAGG GGTATTCCTA    720
   ATTTGCTTTG CACCTTATCA TTTCAGTTTT CCTTTAGATT TCCTGGTGAA GTCCAATGAA    780
   ATTAAAAGCT GCCTAGCCAG AAGGGTGATT CTAATATTTC ATTCTGTGGC ATTGTGTCTT    840
   GCTAGTCTGA ATTCATGTCT TGACCCAGTC ATATACTACT TTTCCACTAA TGAGTTCCGA    900
   AGACGGCTTT CAAGACAAGA TTTGCATGAC AGCATCCAAC TCCATGCAAA ATCCTTTGTG    960
   AGTAACCATA CAGCTTCCAC CATGACACCT GAATTATGC                           999

### (2) INFORMATION FOR SEQ ID NO:6:

(i) SEQUENCE CHARACTERISTICS:
   (A) LENGTH: 60 base pairs
   (B) TYPE: nucleic acid
   (C) STRANDEDNESS: double
   (D) TOPOLOGY: linear
(ii) MOLECULE TYPE: cDNA
(xi) SEQUENCE DESCRIPTION: SEQ ID NO:6:
   CGGATTGTGA ATTTAGTTTT GAACCATCAT TAGGCAAAGA TAGCTTCTCT AGAGAGAATC     60

### (2) INFORMATION FOR SEQ ID NO:7:

(i) SEQUENCE CHARACTERISTICS:
   (A) LENGTH: 554 base pairs
   (B) TYPE: nucleic acid
   (C) STRANDEDNESS: double
   (D) TOPOLOGY: linear
(ii) MOLECULE TYPE: cDNA
(xi) SEQUENCE DESCRIPTION: SEQ ID NO:7:
   TAAAACAAAA AACCAAACTG AATGTGACCT GAAATGCAAG TACATCAGAA CATATCTGCA    60
   ATACCCAAGC CACAGGGAAG AACTTGCAAA ACAACACAGC TTTTCAGTTC TGCTCTATCT   120
   TACTGCTATG GGGAATTCAC TTCTTCAAAG CAGGACCTAT TTGGAGCATT ACGATCCACG   180
   ATTATTGATG TTGACATGTC CATGTAGTAA TTTTTCTTCA AGTCTGTAAA TCTTAAAATA   240
   TCAAATTTCT GTGACATCCT ATAAACATAT GCACTCAACT GTAGTCAGTA CTTTTACCTG   300
   TGAACCTCAG GCACAAAAAG ATTATTAGCT TGGAGTCACC ATAAACATTT AGTTTTGTTG   360
   CAACAGATAC TGAGTCTTTA TGTTCAGAGG AAATGTAAGT GTCTTTTTAT ATTAGTAATC   420
   ACAGTTTACA TGCCATTTTC ATATGTTTGG TTATATTTTA GTGGGATAGA TGATATATTA   480
   CCCTGTGTAA AGAAAATGTA AACATAAGAT CATTTTTATC TCTCAAGTGT GATTACTCTT   540
   CAGAGTTTGA ACCG                                                     554

### (2) INFORMATION FOR SEQ ID NO:8:

(i) SEQUENCE CHARACTERISTICS:
   (A) LENGTH: 36 base pairs
   (B) TYPE: nucleic acid
   (C) STRANDEDNESS: double
   (D) TOPOLOGY: linear
(ii) MOLECULE TYPE: cDNA
(xi) SEQUENCE DESCRIPTION: SEQ ID NO:8:
   CCAGCAGCCA TGGACTACAA GGACGACGAC GACAAA                              36

### (2) INFORMATION FOR SEQ ID NO:9:

(i) SEQUENCE CHARACTERISTICS:
   (A) LENGTH: 20 amino acids
   (B) TYPE: amino acid
   (D) TOPOLOGY: linear
(ii) MOLECULE TYPE: peptide
(xi) SEQUENCE DESCRIPTION: SEQ ID NO:9:
   Trp Phe Leu Met Tyr Pro Phe Arg Phe His Asp Cys Lys Gln Lys Tyr
   1               5                   10                  15
   Asp Leu Tyr Ile
               20

### (2) INFORMATION FOR SEQ ID NO:10:

(i) SEQUENCE CHARACTERISTICS:
   (A) LENGTH: 25 amino acids
   (B) TYPE: amino acid
   (D) TOPOLOGY: linear
(ii) MOLECULE TYPE: peptide
(xi) SEQUENCE DESCRIPTION: SEQ ID NO:10:
   Pro Leu Leu Arg Thr Ser Asp Asp Thr Ser Gly Asn Arg Thr Lys Cys
   1               5                   10                  15
   Phe Val Asp Leu Pro Thr Arg Asn Val
               20                  25

## Description

### Technical Field

This invention relates to a human T-cell receptor. More specifically, this invention relates to nucleotide sequences coding for a human T-cell receptor, to recombinant DNA comprising such nucleotide sequences, vectors comprising such recombinant DNA, and transformed cells comprising such a nucleotide sequence. This invention further relates to polypeptides which function as a receptor in mammalian cells, to antibodies thereto and to ligands which bind to said polypeptide. Further still, this invention relates to an assay for the ability of an agent to act as an agonist or antagonist of said T-cell receptor. Yet further still, this invention relates to antisense sequences to said nucleotide sequences.

### Background Art

G proteins are well known to those skilled in the art as guanine nucleotide-binding proteins. Hence, G-protein coupled receptors are also well known in the art as receptors which are coupled to guanine nucleotide-binding protein. G proteins transmit signals from the associated receptors which are at the cell-surface to certain effector enzymes within the cell via a GTP binding and hydrolysis cycle. G-protein-coupled receptors typically have seven membrane spanning segments. Chaffin, K. E., et al., EMBO Journal 9:3821-3829 (1990) suggest that a pertussis toxin signalling process involves Gᵢ proteins and regulates thymocyte emigration.

Chemofactor response to neutrophil attractant/activation protein-1 (NAP-1) by lymphocytes at an efficacy which is low compared to the neutrophil response is reported by Leonard, E. J., et al., J. Immunol. 144:1323-1330 (1990). The amino acid sequence of NAP-1 has been disclosed by Yoshimura, T., et al., Proc. Natl. Acad. Sci. USA 84:9233-9237 (1987). NAP-1 is also known as interleukin-8 (IL-B).

The cloning and sequences of two G-protein coupled somatostatin receptors (SSTR1 and SSTR2) have been disclosed by Yamada, Y., et al., Proc. Natl. Acad. Sci. USA 89:251-255 (1992). The cloning and sequence of the human C5a G-protein coupled receptor have been disclosed by Gerard, N. P., et al., Biochemistry 29:9274-9281 (1990). The cloning and sequence of a low affinity IL-8 G-protein coupled receptor have been disclosed by Murphy, P. M., et al., Science 253:1280-1283 (1991) and the cloning and sequence of another IL-8 G-protein coupled receptor have been disclosed by Holmes, W. E., et al., Science 253:1278-1280 (1991).

Boulay, F., et al., Biochemistry 29:11123-11133 (1990) disclose the cloning and sequence of two variants of the human N-formylpeptide chemoattractant G-protein coupled receptor.

Polypeptide analogs of an IL-8 receptor-interacting site, antibodies thereto and the use of such antibodies in the immunoassay of IL-8 and to treat inflammation are disclosed in W09204372, published March 19, 1992. The human complement receptor type 1 gene, the encoded protein and fragments thereof, and uses of such proteins and fragments for treatment of immune disorders, myocardial infarct, damage due to inflammation and, in combination with a thrombolytic, thrombosis are disclosed in WO9105047, published April 18, 1991. The preparation and use of a C5a receptor protein is disclosed in EP377489, published July 11, 1990.

### Disclosure of the Invention

This invention provides a T-cell receptor and nucleotide sequences encoding the receptor. The nucleotide sequences of this invention consist essentially of the nucleotide sequences shown hereinbelow as SEQUENCE I.D. NO: 1 and SEQUENCE I.D. NO: 5. SEQUENCE I.D. NO: 1 comprises the nucleotide sequence of SEQUENCE I.D. NO: 5. Preferred are the nucleotide sequences of SEQUENCE I.D. NO: 1 and SEQUENCE I.D. NO: 5.

This invention further provides recombinant DNA comprising a nucleotide sequence essentially of SEQUENCE I.D. NO: 1 or SEQUENCE I.D. NO: 5. Preferred is such recombinant DNA wherein the nucleotide sequence is functionally linked to a promoter.

This invention still further provides vectors comprising recombinant DNA as hereinabove described.

Also provided by this invention are transformed or transfected host cells comprising a nucleotide sequence essentially of SEQUENCE I.D. NO: 1 or SEQUENCE I.D. NO: 5. The transformed or transfected host cells of this invention are all cells suitable for the replication and/or expression of the nucleotide sequences hereinabove described. By way of example and not of limitation such cells include Escherichia coli, Saccharomyces cerevesiae, Yarrowia lipolytica and mammalian cells.

This invention further provides antisense sequences to nucleotide sequences which are essentially SEQUENCE I.D. NO: 1 or SEQUENCE I.D. NO: 5. The term expression sequence when used in the singular or plural includes, but is not limited to the nucleotide sequences which code for the translational controls and/or the structural sequence. The antisense sequences of this invention are useful to down-regulate the expression of polypeptide SEQUENCE I.D. NO: 2 in cells and, thus, could find usefulness in treating graft rejection and/or inflammatory conditions in an animal, including a human being.

This invention also provides a substantially pure polypeptide of SEQUENCE I.D. NO: 2. The polypeptide of SEQUENCE I.D. NO: 2 is encoded by the nucleotide sequence of SEQUENCE I.D. NO: 5 as well as SEQUENCE I.D. NO: 1 and is a putative human T-cell G-protein coupled receptor. The polypeptides of this invention as hereinabove described are useful for preparing antibodies thereto. Further, the polypeptides are useful in assay procedures such as those described hereinbelow for agonists or antagonists of a T-cell receptor. Still further, the polypeptides of this invention comprising a binding site are useful in the preparation of soluble polypeptides or fragments thereof which inhibit receptor function by competing for binding to the ligand. The polypeptides of this invention are also useful in the isolation of a ligand of the receptor formed by the polypeptide of SEQUENCE I.D. NO: 2 as well as identification of clones expressing a natural ligand thereof from within an expression library.

This invention also provides methods to assay an agent for the ability thereof to act as a ligand or an agonist of a T-cell receptor comprising a polypeptide of SEQUENCE I.D. NO: 2; methods to assay an agent for the ability thereof to act as a selective antagonist of such a T-cell receptor; and methods to assay an agent for the ability thereof to act as an antagonist of such T-cell receptors. The methods are set forth hereinbelow.

Also provided by this invention are the antibodies herein described which are useful for identifying cells expressing the receptor formed by the polypeptide of SEQUENCE I.D. NO: 2 and could find usefulness in treating graft rejection and/or inflammatory conditions in an animal, including a human being. Preferably the antibodies are monoclonal antibodies.

Still further, this invention provides a ligand to the receptor which ligand is useful in a receptor binding assay to identify a receptor antagonist or agonist.

As used throughout this Specification and the appendant claims, "recombinant DNA" means DNA which comprises a nucleotide sequence according to this invention regardless of the nature of the DNA. Thus, by way of example and not of limitation, recombinant DNA includes linear DNA fragments as well as chromosomal DNA wherein a nucleotide sequence according to this invention has been inserted as well as derivatives by replication thereof.

### Description of the Drawing

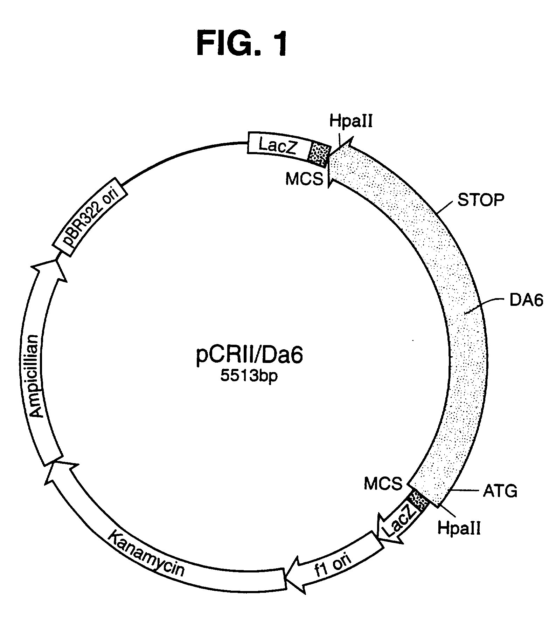

Figure 1 is a map of plasmid pCR11/Da6 wherein pBR322 ori represents the origin of replication from plasmid pBR322, Ampicillin represents the coding sequence for ampicillin resistance, Kanamycin represents the coding sequence for Kanamycin resistance, f1 ori represents the origin of replication from phage f1, Lac Z represents the partial coding regions for β-galactosidase, MCS represents a multiple cloning site, DA6 represents the sequence containing SEQUENCE I.D. NO: 1 as described herein, Hpall represent the restriction site locations for the enzyme Hpall, ATG represents the location of the start codon for the coding sequence of Da6 (i.e., SEQUENCE I.D. NO: 5) and STOP represents the location of the stop codon at the 3'terminus of the coding sequence of Da6 (i.e., SEQUENCE I.D. NO: 5).

### Detailed Description

### 1. PREPARATION AND IDENTIFICATION OF CLONE Da6

Employing the guanidium isothiocyanate method described by Chomczynski, P., et al., Analytical Biochemistry 162: 156-159 (1987), mRNA was isolated from fresh human peripheral blood T cells. According to methods well known to those skilled in the art, and as described by Molecular Cloning, Second Edition, Part 2, Sambrook, J., et al., 1989, Cold Spring Harbor Laboratory Press, Cold Spring Harbor, New York, pp. 8.60-8.63, first strand cDNA was generated from the mRNA using reverse transcriptase. Two primers were commercially prepared by Genosys Corp, Houston, TX. One primer was a degenerate oligonucleotide corresponding to a consensus sequence obtained by comparison of the M2 transmembrane domain of the human interleukin 8 [IL-8, Holmes, W. E., et al., Science 253:1278-1280 (1991)], F-met-Leu-Phe [FMLP, Boulay, F., et al., Biochemistry 29:11123-11133 (1990)], complement C5a [Gerard, N.P., et al., Nature 349:614-617 (1991)], neurokinin A [NKA, Gerard, N.P., et al., The Journal of Biological Chemistry 265:20455-20462 (1990)], and glucocorticoid-induced receptor [MUSGCP, Harrigan, M.T., et al., Molecular Endocrinology 5:1331-1338 (1991)]. The M2 degenerate primer was of the sequence ATAAGCTTAANNTNGCNNTNGCNGAC (SEQUENCE I.D. NO: 3). The second primer was a degenerate oligonucleotide corresponding to a consensus sequence obtained by comparison of the M6 transmembrane domain of an IL-8, hFMLP, hC5a, hNKA and MUSGCP receptor. The M6 degenerate primer (reverse complement) was of the sequence TCGAATTCNANNTNNTANGGNNNCCA (SEQUENCE I.D. NO: 4).

Using the M2 and M6 degenerate primers of SEQUENCE I.D. NO: 3 and SEQUENCE I.D. NO: 4, the cDNA prepared as described above was amplified using a modified PCR amplification protocol whereby the DNA was amplified 30 cycles with a 30-second denaturization temperature of 94° C and a 45-second elongation temperature of 72°C. The annealling temperature was 37°C for the first cycle and the temperature was increased 0.5°C per cycle thereafter. The resulting amplified sequences were cloned into plasmid pBSK (Stratogene, LaJolla, CA) as a restriction fragment (EcoRI/Hindlll). Resulting clones were sequenced using automated fluorescent sequencing on Applied Biosystems 373A Automated DNA sequences (Applied Biosystems Inc. Foster City, CA).

A partial Da6 clone was identified by comparison of the translated sequence of the clones to the PIR protein database using BLAST sequence comparison software [National Center for Biotechnology Information, National Library of Medicine, NIH, Bethesda, MD; Altschul, S.F., et al., Joumal of Molecular Biology 215:403-410 (1990)]. The partial Da6 clone was then used to isolate the complete Da6 clone by hybridization to a human thymus λ-gt-11 cDNA library (Clontech, Palo Alto, CA). Initially, about 500,000 plaques, immobilized on nitrocellulose filters, were hybridized to the partial Da6 clone in 5X Denhardt's solution, 5X SSPE, 50% formamide, 100ug/ml ssDNA, 0.1% SDS, and 10% Dextran sulfate at 42°C for 16 hours. The filters then were washed in 0.16X SSC/1.0% SDS at 50°C for 30 minutes. Then, the filters were autoradiographed to X-ray film, with intensifying screens, for 12 hours at -70°C. Resulting positive clones were subjected to two more rounds of plaque screening as described above using the partial Da6 cDNA to purify the full length Da6 clone. A resulting positive clone was amplified and its DNA was recovered as outlined in Molecular Cloning, Second Edition, Part 1, Sambrook, et al., Op. cit., pp. 2.69-2.71. Purified DNA was then subjected to PCR (Hoffman LaRoche, Nutley, NJ) using commercially available DNA primers that flank the cloning region of λgt11 (Clonetech, Palo Alto, CA). The amplified sequences were then cloned into pCRII - 'TA Cloning System' (Invitrogen, San Diego, CA) using the ligation conditions supplied by the manufacturer. The full length Da6 clone was verified by fluorescent automated sequencing (Applied Biosystems Inc., Foster City, CA). Clone Da6 contains a unique sequence based on searches of both the PIR and Genbank sequence databases (Intelligenetics, Inc., Mountain View, CA). Due to certain homology between Da6 and the platelet activating factor and IL-8 receptors, it was concluded that Da6 encodes a novel putative G-protein coupled receptor. A map of plasmid pCRII/Da6 is presented in Figure 1.

Using Da6 as a probe, it was shown by Northem analysis (Molecular Cloning, Second Edition, Part 1, Sambrook, J., et al., Op. cit., pp 7.37-7.52) that the receptor encoded thereby is preferentially expressed in T cells. The nucleotide sequence of the receptor of clone Da6, including its associated 5' and 3' nucleotide sequences, is shown below as SEQUENCE I.D. NO: 1.

Clone Da6 which comprises SEQUENCE I.D. NO: 1 in pCRII (Invitrogen, San Diego, CA) has been deposited under the terms of the Budapest Treaty in the American Type Culture Collection, Rockville, MD, a recognized depository affording permanence of the deposits and ready accessibility thereto by the public if a patent is granted on this application. Clone Da6 comprises Escherichia coli transformed with and containing plasmid pCRII/Da6. The Hpall fragment of pCRII/Da6 contains 999bp of Da6 coding sequence (SEQUENCE I.D. NO: 5), 60 bp of Da6 5' flanking sequence (SEQUENCE I.D. NO: 6), and 554 bp of Da6 3' flanking sequence (SEQUENCE I.D. NO: 7). The deposit is made available during the pendency of this application to one determined by the Commissioner of the United States Patent and Trademark Office to be entitled thereto under 37 C.F.R. 1.14 and 35 U.S.C. 122, and in accordance with foreign patent laws in countries wherein counterparts of this application, or its progency, are filed. All restrictions on the availability to the public of the microorganism deposited will be irrevocably removed upon granting of the patent. Clone Da6 has been assigned deposit number ATCC 69248.

### 2. EXPRESSION OF SEQUENCE I.D. NO: 1 IN MAMMALIAN CELLS

The full length nucleotide sequence of SEQUENCE I.D. NO: 1 is excised from pCRII/Da6 using restriction enzyme Hpall and is blunt end cloned into the Eco RV restriction sites of vector pcDNA/Neo (Invitrogen, San Diego, CA). For purposes of subsequent antibody recognition, a synthetic flag sequence, CCAGCAGCCATGGACTACAAGGACGACGACGACAAA (SEQUENCE I.D. NO: 8), is inserted at the 5' end of SEQUENCE I.D. NO: 1 in vector pcDNA/Neo by ligation of annealed complementary oligonucleotides containing this sequence in translational frame upstream of the Da6 coding sequence (Harlow, E., et al., Antibodies, 1988, Cold Spring Harbor Laboratory, New York). The vector is then amplified in transformed, competent E. coli cells and purified from those cells using CsCI gradient purification twice (Molecular Cloning, Second Edition, Part 1, Sambrook, J., et al., Op. cit., pp. 1.42-1.43). Appropriate mammalian host cells such as β cells, fibroblasts or COS cells (i.e., cells which do not naturally express a protein of SEQUENCE I.D. NO: 2) are transfected with 10-50 µg of the purified vector using calcium chloride, electroporation, liposomes or other means well known to those skilled in art (Molecular Cloning, Second Edition, Part 3, Sambrook, J. et al., Op. cit., pp. 16.30-16.55). The resulting transfected cells are tested by Northem analysis or reverse transcription polymerase chain reaction (RT-PCR) of isolated mRNA for expression of mRNA corresponding to the flagged polypeptide. Expression of SEQUENCE I.D. NO: 2 from SEQUENCE I.D. NO: 1 is detected using an antibody to the FLAG sequence using FACS, immunoprecipitation, Western blotting or immunohistochemical staining. Such techniques are well known to those skilled in the art and are described in Molecular Cloning, Second Edition, Part 3, Sambrook, J. et al., Op. cit. pp. 18.19-18.75 and Kunz, D., et al., J. Biol. Chem. 267: 9101-9106 (1992). Alternatively, an antibody to an extracellular sequence of SEQUENCE I.D. NO: 2 can be used to detect expression according to the techniques described immediately above.

### 3. PRODUCTION OF ANTIBODIES TO SEQUENCE I.D. NO: 2

Polypeptides corresponding to unique sequences formed within putative extracellular domains of SEQUENCE I.D. NO: 2 [i.e., WFLMYPFRFHDCKQKYDLYI (SEQUENCE I.D. NO: 9) and PLLRTSDDTSGNRTKCFVDLPTRNV (SEQUENCE I.D. NO: 10)] are synthesized and conjugated to an antigen such as key hole limpet hemocyanin (KLH) or bovine serum albumin (BSA). The conjugates are resuspended in an adjuvant and used to immunize and boost animals and sera is drawn after each boost. The sera is tested using an ELISA for the ability to bind the conjugated peptide but not a control peptide. Sera that reacts to the conjugated peptide is then tested for the ability to bind to the surface of SEQUENCE I.D. NO: 2 expressing cells using FACS, ELISA, immunoprecipitation, or Western blotting or immunocytohistochemistry. Alternatively, fusion proteins are prepared using polypeptide sequences from SEQUENCE I.D. NO: 2 and used to immunize animals to produce antisera. To produce such fusion proteins, nucleotide sequences from SEQUENCE I.D. NO: 1 or SEQUENCE I.D. NO: 5 can be ligated to lacZ DNA or other DNA and expressed in E. coli. The fusion protein is then purified and used to immunize animals as described above.

Alternatively and preferably, monoclonal antibodies to the appropriate extracellular polypeptide sequence are prepared according to standard techniques (Harlow, E., et al, Antibodies, 1988, pp. 55-240, Cold Spring Harbor Laboratory, NY). Polypeptides or fusion proteins containing peptide sequences from SEQUENCE I.D. NO: 2 are used to immunize mice. An appropriate amount of antigen peptides or fusion proteins (5-50 µg) are mixed with complete adjuvant and injected into the intraperitoneal cavity of mice. Alternatively, the antigen may be delivered by subcutaneous injection or by injection directly into lymphoid organs. Subsequent injections are made using incomplete Freund's adjuvant. The sera is tested using an ELISA for the abilty to bind to the appropriate peptide antigen but not a control peptide antigen. Sera that reacts to the appropriate antigen is then tested for the ability to bind to the surface of SEQUENCE I.D. NO: 2 expressing cells using FACS, ELISA, immunoprecipitation or immunocytochemistry. Animals that produce antisera that reacts with the appropriate antigen are used to make hybridomas producing monoclonal antibodies. Animals are boosted with the appropriate antigen 3-5 days prior to fusion. Splenocytes are obtained from the mice and fused to suitable myeloma cells using polyethylene glycol (Harlow, E., et al., Antibodies, Ibid.). The hybridomas are selected for using hypoxanthine, aminoptesin and thymidine Selection (HAT) medium or other suitable selection medium. Hybridoma pools are tested for antibody production using ELISA assay. Positive pools are cloned and individual clones are similarly tested for production of appropriate antibodies. Positive clones are expanded and grown to produce supematants containing the monoclonal antibody to SEQUENCE I.D. NO: 2. The monoclonal antibodies may be purified on protein A columns (Harlow, E., et al., Antibodies, Op. cit.) or by other methods known to those skilled in the art of protein purification.

### 4. DETECTION OF Da6 FUNCTION BY CALCIUM FLUX ASSAY

A. Fura-2 assay. Essentially SEQUENCE I.D. NO: 1 or SEQUENCE I.D. NO: 5 transfected cells are incubated with fura-2 acetoxymethyl ester (2.5µM) in calcium/magnesium-free PBS for 30 min. at 37°C. Then, the cells are washed, resuspended in calcium/magnesium-free PBS containing 10mM HEPES pH7.4, 0.25% BSA and 10mM glucose. The cells are dispersed into quartz cuvettes and the extemal calcium concentration is adjusted to 1mM with CaCl₂. Receptor ligand is added at an effective concentration and fluorescence is monitored using a fluorescence spectrophotometer at excitation wavelengths of 340 nm and 380 nm and emission wavelength of 510nm. Calcium levels are measured using the ratio of the resulting fluorescence readings.
B. Flow cytometry. According to the method described by Holmes, W. E. et al., Science 253: 1278-1280 (1991), cells transfected with essentially SEQUENCE I.D. NO: 1 or SEQUENCE I.D. NO: 5 are incubated with indo-1 acetoxymethyl ester (2µM) in RPMI for 30 min. at 37°C. The entracellular calcium is then measured with a flow cytometer from the ratio of fluorescence at wavelengths of 405 nm and 525 mm.

### 5. DETECTION OF Da6 FUNCTION BY REPORTER ASSAY

Transfection competent cells are co-transfected using electroporation or other transfection protocols (Molecular Cloning, Second Edition, Part 3, Sambrook, J., et al., Op. cit., pp. 16.30-16.55) with a vector capable of expressing SEQUENCE I.D. NO: 2 in said cells and a vector encoding luciferase under control of a G-protein sensitive promoter (e.g., minimal multimerized CRE, NFkB or minimal multimerized AP-1 promoter driving luciferase as CAT). Minimal multimerized CRE, NFKB or AP-1 promoter luciferase/CAT plasmids are derived by cloning a ligated double strand sequence containing multiple copies of the CRE, NFKB or AP-1 consensus sequence binding sites upstream of a minimal thymidine kinase or IL-2 promoter. The co-transfected cells are stimulated with an effective amount of receptor ligand and functioning of the SEQUENCE I.D. NO: 2 receptor is measured by the amount of luciferase or CAT activity produced by the cell as described in Molecular Cloning, Second Edition, Part 3, Sambrook, J. et al., Op. cit., pp. 16.60-16.65, and by DeWet, J.R., et al., Molecular and Cellular Biology 7:725-737 (1987).

### 6. PROCESS FOR IDENTIFICATION OF NATURAL UGANDS AND AGONISTS OF SEQUENCE I.D. NO: 2

Employing transfected cells according to the Fura-2, flow cytometry or reporter assay described hereinabove and incubating the cells in the presence of a chemical entity in an appropriate solubilized form, a natural product, a purified factor or a crude cellular extract at varying concentrations and assaying for the effect thereof upon said cells when compared to non-transfected cells enables selection of agonists and natural ligands of SEQUENCE I.D. NO: 2 as those entities which stimulate calcium flux (Fura-2 or flow cytometry assay) or increase luciferase/CAT activity (reporter assay).

### 7. IDENTIFICATION OF SELECTIVE SEQUENCE I.D. NO: 2 ANTAGONISTS

Cells, stably transfected with and capable of expressing essentially SEQUENCE I.D. NO: 1 or SEQUENCE I.D. NO: 5 are incubated in the presence of a defined concentration of a chemical entity prior to the addition thereto of a natural ligand or agonist of SEQUENCE I.D. NO: 2. In order to assay for selectivity of the antagonist, a known G-protein coupled receptor containing cell line (i.e., an IL-8R expressing control cell line) is also preincubated with the chemical entity prior to addition of a natural ligand (e.g. IL-8) or agonist thereof. In both cases, the effect of the entity on either calcium flux or reporter activity is determined. Those chemical entities which inhibit or block ligand or agonist activation of calcium flux in the stably transfected cells but not in the control cell line are antagonists selective for SEQUENCE I.D. NO: 2.

Alternatively, a receptor binding assay as described in any one of Kunz, D., et al., J. Biol. Chem. 267: 9101-9106 (1992), Nourshargh, S., et al., J. Immunol. 148: 106-111 (1992), Holmes, W.E., et al., Science 253: 1278-1280 (1991) or Gerard, N.P., et al., Biochemistry 29: 9274-9281 (1990) employing a labeled ligand of SEQUENCE I.D. NO:2 can be employed. Transfected cells capable of expressing essentially SEQUENCE I.D. NO: 1 or SEQUENCE I.D. NO: 5 are pre-incubated with varying concentrations of a chemical entity for a pre-determined period of time under appropriate binding conditions followed by the addition thereto of a labeled ligand that binds to SEQUENCE I.D. NO: 2. As a control, the binding assay is also performed using the same chemical entity but with a control receptor (e.g. IL-8R) transfected into the same host cell line as was used to obtain the essentially SEQUENCE I.D. NO: 1 or SEQUENCE I.D. NO: 5 transfected cells and with a labeled ligand of the control receptor. A chemical entity which blocks or inhibits the binding of the labeled ligand to the essentially SEQUENCE I.D. NO: 1 or SEQUENCE I.D. NO: 5 transfected cells but not control cells is an antagonist selective for SEQUENCE I.D. NO: 2.

### 8. TRANSGENIC ANIMALS COMPRISING SEQUENCE I.D. NO: 1 OR SEQUENCE I.D. NO: 5 (not claimed)

A transgenic vector is constructed comprising essentially SEQUENCE I.D. NO: 1 or SEQUENCE I.D. NO: 5 together with transcriptional and processing elements to allow expression in the tissue or tissues of choice in the transgenic animal. Expression may be directed to T lymphocytes using vectors described [Cooke, M.P. et al., Cell 65:281-291 (1991)]. Once constructed and amplified by methods well known to those skilled in art, approximately 200 copies of linear vector DNA are microinjected into the male pronucleus of the fertilized egg of the animal to be made transgenic according to the methods described by Hanahan, D., Science 246: 1265-1275 (1989) and Brinster, R. L., et al., The Harvey Lecture Series 80: 1-38 (1986). The injected eggs are then implanted into the oviduct of pregnant foster mothers and the offspring are tested for transgene integration using dot blot analysis, Southem analysis or PCR analysis as described in Molecular Cloning, Second Edition, Part 2, Sambrook, J., et al., Op. cit., pp. 9.4-9.58 and 14.5-14.21. Tissue from animals positive for transgene integration is tested for transgene expression using Northern analysis or PCR analysis for detection of transgene mRNA levels. Transgene positive animals that express the transgene are propagated and the offspring thereof are tested for transgene presence and function to verify establishment of a transgenic animal line. Such transgenic animals are useful for the generation of animal disease models resulting from overexpression of receptors from SEQUENCE I.D. NO: 2. These animals are also useful for testing the effects of antagonists, agonists or natural ligands on receptor function in vivo.

### 9. ANTISENSE SEQUENCES

A synthetic antisense DNA sequence of approximately 15 to 30 base pairs in length and complementary to a portion of an expression sequence of essentially SEQUENCE I.D. NO: 1 or SEQUENCE I.D. NO: 5. The antisense sequence is prepared by standard methods well known to those skilled in the art such as by the use of a DNA synthesizer (Applied Biosystems, Inc., Mountain View, CA). The antisense sequence is then administered to a T-cell or animal at an amount effective to inhibit expression of SEQUENCE I.D. NO: 2. Further, modified oligonucleotides which bind to the antisense sequences and result in increased cellular uptake, in vivo absorption, bioavailability and/or protection from nucleases can be prepared and employed in compositions with such antisense sequences according to methods described by Cohen, J.S., Pharmac. Ther. 52: 211-225 (1991).

## Claims

1. A nucleotide sequence encoding a human T-cell receptor consisting essentially of SEQUENCE I.D. NO: 1.

2. Recombinant DNA comprising a nucleotide sequence according to claim 1.

3. Recombinant DNA according to claim 2, wherein the nucleotide sequence is functionally linked to a promoter.

4. A vector comprising recombinant DNA according to claim 2 or claim 3.

5. A transformed or transfected host cell comprising a nucleotide sequence according to claim 1.

6. A nucleotide sequence encoding a human T-cell receptor consisting essentially of SEQUENCE I.D. NO: 5.

7. Recombinant DNA comprising a nucleotide sequence according to claim 6.

8. Recombinant DNA according to claim 7, wherein the nucleotide sequence is functionally linked to a promoter.

9. A vector comprising recombinant DNA according to claim 7 or claim 8.

10. A transformed or transfected host cell comprising a nucleotide sequence according to claim 6.

11. Escherichia coli ATCC 69248.

12. Plasmid pCRII/Da6, illustrated graphically in Figure 1.

13. A substantially pure human T-cell receptor polypeptide of SEQUENCE I.D. NO: 2.

14. An antibody to a polypeptide according to claim 13.

15. An antibody according to claim 14. wherein the antibody is a monoclonal antibody.

16. A method to assay an agent for the ability thereof to act as a ligand or an agonist of a cell receptor comprising a polypeptide according to claim 13, which method comprises:
(a) incubating cells transfected with and capable of expressing SEQUENCE I.D. NO: 1 or SEQUENCE I.D. NO: 5, in the presence of a solubilized chemical entity, a natural product. a purified factor or a crude cellular extract;
(b) incubating control cells of the same type as in step (a), but which are not so transfected, in the presence of the same solubilized chemical entity, natural product, purified factor or crude cellular extract as employed in step (a);
(c) assaying the effect of the solubilized chemical entity, natural product, purified factor or crude cellular extract on the cells of steps (a) and (b); and
(d) determining whether the solubilized chemical entity, natural product, purified factor or crude cellular extract increase or stimulate activity of the cell receptor comprising a polypeptide according to claim 13 for the cells of step (a) when compared to the cells of step (b).

17. The method according to claim 16 wherein, in step (c), the effect is assayed by measuring calcium flux.

18. The method according to claim 16 wherein, in step (a), the transfected cells are co-transfected with a vector encoding luciferase under control of a G-protein sensitive promoter; in step (b), the cells are transfected with the vector containing luciferase under control of G-protein sensitive promoter which is used to co-transfect the cells of step (a); and, in step (c), the effect is assayed by measuring luciferase activity.

19. A method to assay an agent for the ability thereof to act as a selective antagonist of a cell receptor comprising a polypeptide according to claim 13, which method comprises:
(a) separately incubating cells transfected with and capable of expressing SEQUENCE I.D. NO: 1 or SEQUENCE I.D. NO: 5, and cells containing a known G-protein coupled receptor in the presence of a chemical entity;
(b) adding to the incubations of step (a) a natural ligand or agonist of SEQUENCE I.D. NO: 2 to the stably transfected cells, and a natural ligand or agonist to the known G-protein coupled receptor cells;
(c) assaying the effect of the chemical entity on the cells; and
(d) determining whether the chemical entity selectively inhibits or blocks the natural ligand or agonist of SEQUENCE I.D. NO: 2.

20. The method according to claim 19 wherein, in step (c), the effect is assayed by measuring calcium flux.

21. The method according to claim 19 wherein. in step (a), the cells are co-transfected and the cells containing a known G-protein coupled receptor are transfected with a vector encoding luciferase under control of a G-protein sensitive promoter; and, in step (c), the effect is assayed by measuring luciferase activity.

22. A method to assay an agent for the ability thereof to act as an antagonist of a cell receptor comprising a polypeptide according to claim 13, which method comprises:
(a) separately incubating transfected cells capable of expressing SEQUENCE I.D. NO: 1 or SEQUENCE I.D. NO: 5, and control cells of the same type but transfected with and capable of expressing a control receptor other than a receptor comprising a polypeptide according to claim 13 with varying concentrations of a chemical entity;
(b) adding to the respective cells of step (a) a labelled ligand of the respective receptors thereof; and
(c) determining whether the chemical entity blocks or inhibits the binding of the labelled ligand to expressing SEQUENCE I.D. NO: 1 or SEQUENCE I.D. NO: 5 transfected cells but not to the control cells.

23. An antisense sequence to an expression sequence of a nucleotide sequence according to claim 1 or claim 6.

## Patentansprüche

1. Nucleotidsequenz, die einen Human-T-Zellrezeptor kodiert, im wesentlichen bestehend aus Sequenz ID Nr. 1.

2. Rekombinante DNA enthaltend eine Nucleotidsequenz nach Anspruch 1.

3. Rekombinante DNA nach Anspruch 2, worin die Nucleotidsequenz funktionell mit einem Promotor verbunden ist.

4. Vektor enthaltend rekombinante DNA nach Anspruch 2 oder Anspruch 3.

5. Transformierte oder transfizierte Wirtszelle umfassend eine Nucleotidsequenz nach Anspruch 1.

6. Nucleotidsequenz, die einen humanen T-Zellrezeptor kodiert, im wesentlichen bestehend aus Sequenz ID Nr. 5.

7. Rekombinante DNA enthaltend eine Nucleotidsequenz nach Anspruch 6.

8. Rekombinante DNA nach Anspruch 7, worin die Nucleotidsequenz funktionell mit einem Promotor verbunden ist.

9. Vektor enthaltend rekombinante DNA nach Anspruch 7 oder Anspruch 8.

10. Transformierte oder transfizierte Wirtszelle enthaltend eine Nucleotidsequenz nach Anspruch 6.

11. Escherichia coli ATCC 69248.

12. Plamid pCRII/Da6, graphisch dargestellt in Figur 1.

13. Im wesentlichen reines humanes T-Zellrezeptorpolypeptid mit Sequenz ID Nr. 2.

14. Antikörper gegen ein Polypeptid nach Anspruch 13.

15. Antikörper nach Anspruch 14, worin der Antikörper ein monoklonaler Antikörper ist.

16. Verfahren zum Nachweis der Fähigkeit eines Mittels, als Ligand oder Agonist eines Zellrezeptors zu wirken, umfassend ein Polypeptid nach Anspruch 13, wobei das Verfahren umfaßt, daß man:
(a) Zellen, die mit Sequenz ID Nr. 1 oder Sequenz ID Nr. 5 transfiziert sind und diese Sequenzen exprimieren können, in Gegenwart einer solubilisierten chemischen Substanz, einem natürlichen Produkt, einem gereinigten Faktor oder einem rohen Zellextrakt inkubiert;
(b) Kontrollzellen der gleichen Art, wie in Stufe (a), die aber nicht transfiziert sind, in Gegenwart der gleichen solubilisierten chemischen Substanz, des natürlichen Produkts, gereinigten Faktors oder rohen Zellextraktes, wie er in Stufe (a) angewendet wird, inkubiert;
(c) die Wirkung der solubilisierten chemischen Substanz, des natürlichen Produkts, des gereinigten Faktors, oder rohen Zellextraktes auf die Zellen der Stufen (a) und (b) untersucht und
(d) bestimmt, ob die solubilisierte chemische Substanz, das natürliche Produkt, der gereinigte Faktor oder der rohe Zellextrakt die Aktivität des Zellrezeptors, der ein Polypeptid nach Anspruch 13 umfaßt, erhöht oder stimuliert für die Zellen in Stufe (a) verglichen mit den Zellen von Stufe (b).

17. Verfahren nach Anspruch 16, worin in Stufe (c) die Wirkung untersucht wird, indem der Calciumflux gemessen wird.

18. Verfahren nach Anspruch 16, worin in Stufe (a) die transfizierten Zellen mit einem Vektor co-transfiziert werden, der Luciferase unter der Kontrolle eines für G-Protein empfindlichen Promotors codiert; in Stufe (b) die Zellen mit dem Vektor, der Luciferase unter der Kontrolle eines für G-Protein empfindlichen Promotors enthält, der verwendet wird, um die Zellen in Stufe (a) zu cotransfizieren, transfiziert werden und in Stufe (c) die Wirkung getestet wird, indem die Luciferaseaktivität gemessen wird.

19. Verfahren, um die Fähigkeit eines Mittels zu testen, als selektiver Antagonist eines Zellrezeptors zu wirken, der ein Polypeptid nach Anspruch 13 umfaßt, wobei das Verfahren umfaßt, daß man:
(a) getrennt Zellen, die mit Sequenz ID Nr. 1 oder Sequenz ID Nr. 5 transfiziert sind oder diese Sequenzen exprimieren können, und Zellen, die einen bekannten mit G-Protein gekoppelten Rezeptor enthalten, in Gegenwart einer chemischen Substanz inkubiert;
(b) zu den Inkubationen von Stufe (a) einen natürlichen Liganden oder Agonisten von Sequenz ID Nr. 2 zu den stabil transfizierten Zellen, und einen natürlichen Liganden oder Agonisten zu den bekannten mit G-protein gekoppelten Rezeptorzellen zugibt;
(c) die Wirkung der chemischen Substanz auf die Zellen untersucht und
(d) bestimmt, ob die chemische Substanz selektiv den natürlichen Liganden oder Agonisten von Sequenz ID Nr. 2 hemmt oder blockiert.

20. Verfahren nach Anspruch 19, worin in Stufe (c) die Wirkung bestimmt wird, indem der Calciumflux gemessen wird.

21. Verfahren nach Anspruch 19, worin in Stufe (a) die Zellen cotransfiziert werden und die Zellen, die einen bekannten mit G-Protein gekoppelten Rezeptor enthalten, mit einem Vektor transfiziert werden, der Luciferase unter der Kontrolle eines für G-Protein empfindlichen Promotors codiert, und in Stufe (c) die Wirkung untersucht wird, indem die Luciferaseaktivität gemessen wird.

22. Verfahren um die Fähigkeit eines Mittels zu untersuchen, als Antagonist eines Zellrezeptors, der ein Polypeptid nach Anspruch 13 umfaßt, zu wirken, wobei das Verfahren umfaßt, daß man:
(a) transfizierte Zellen, die Sequenz ID Nr. 1 oder Sequenz ID Nr. 5 exprimieren können, und Kontrollzellen der gleichen Art, die aber mit einem anderen Kontrollrezeptor, als dem Rezeptor, der ein Polypeptid nach Anspruch 13 umfaßt, transfiziert sind und diesen exprimieren können, getrennt mit verschiedenen Konzentrationen einer chemischen Substanz inkubiert;
(b) zu den jeweiligen Zellen von Stufe (a) einen markierten Liganden der jeweiligen Rezeptoren zugibt und
(c) bestimmt, ob die chemische Substanz die Bindung des markierten Liganden an Sequenz ID Nr. 1 oder Sequenz ID Nr. 5 exprimierende transfizierte Zellen, nicht aber Kontrollzellen blockiert oder hemmt.

23. Antisense-Sequenz einer Expressionssequenz einer Nucleotidsequenz nach Anspruch 1 oder Anspruch 6.

## Revendications

1. Séquence de nucléotides codant pour un récepteur de lymphocytes T humains, consistant essentiellement en la SEQUENCE I.D. N° 1.

2. ADN recombinant comprenant une séquence de nucléotides suivant la revendication 1.

3. ADN recombinant suivant la revendication 2, dans lequel la séquence de nucléotides est liée fonctionnellement à un promoteur.

4. Vecteur comprenant l'ADN recombinant suivant la revendication 2 ou la revendication 3.

5. Cellule-hôte transformée ou transfectée comprenant une séquence de nucléotides suivant la revendication 1.

6. Séquence de nucléotides codant pour un récepteur de lymphocytes T humains consistant essentiellement en la SEQUENCE I.D. N° 5.

7. ADN recombinant comprenant une séquence de nucléotides suivant la revendication 6.

8. ADN recombinant suivant la revendication 7, dans lequel la séquence de nucléotides est liée fonctionnellement à un promoteur.

9. Vecteur comprenant l'ADN recombinant suivant la revendication 7 ou la revendication 8.

10. Cellule-hôte transformée ou transfectée comprenant une séquence de nucléotides suivant la revendication 6.

11. Escherichia coli ATCC 69248.

12. Plasmide pCRII/Da6, représenté graphiquement sur la figure 1.

13. Polypeptide récepteur de lymphocytes T humains essentiellement pur, ayant la SEQUENCE I.D. N° 2.

14. Anticorps contre un polypeptide suivant la revendication 13.

15. Anticorps suivant la revendication 14, qui est un anticorps monoclonal.

16. Méthode d'analyse d'un agent pour déterminer son aptitude à jouer le rôle de ligand ou d'agoniste d'un récepteur cellulaire, comprenant un polypeptide suivant la revendication 13, méthode qui comprend les étapes consistant :
(a) à mettre en incubation des cellules transfectées avec et capables d'exprimer la SEQUENCE I.D. N° 1 ou la SEQUENCE I.D. N° 5, en présence d'une entité chimique solubilisée, un produit naturel, un facteur purifié ou un extrait cellulaire brut ;
(b) à mettre en incubation des cellules témoins du même type que dans l'étape (a), mais qui n'ont pas subi une telle transfection, en présence d'une entité chimique solubilisée, d'un produit naturel, d'un facteur purifié ou d'un extrait cellulaire brut identique à celui utilisé dans l'étape (a) ;
(c) à analyser l'effet de l'entité chimique solubilisée, du produit naturel, du facteur purifié ou de l'extrait cellulaire brut sur les cellules des étapes (a) et (b) ; et
(d) à déterminer si l'entité chimique solubilisée, le produit naturel, le facteur purifié ou l'extrait cellulaire brut augmente ou stimule l'activité du récepteur cellulaire comprenant un polypeptide suivant la revendication 13 pour les cellules de l'étape (a) comparativement aux cellules de l'étape (b).

17. Méthode suivant la revendication 16, dans laquelle, dans l'étape (c), l'effet est analysé par mesure du flux de calcium.

18. Méthode suivant la revendication 16, dans laquelle, dans l'étape (a), les cellules transfectées sont co-transfectées avec un vecteur codant pour la luciférase ou sous le contrôle d'un promoteur sensible à la protéine G ; dans l'étape (b), les cellules sont transfectées avec le vecteur contenant de la luciférase sous le contrôle d'un promoteur sensible à la protéine G qui est utilisé pour co-transfecter les cellules de l'étape (a) ; et, dans l'étape (c), l'effet est analysé par mesure de l'activité de la luciférase.

19. Méthode pour analyser un agent afin de déterminer son aptitude à jouer le rôle d'antagoniste sélectif d'un récepteur cellulaire comprenant un polypeptide suivant la revendication 13, méthode qui comprend les étapes consistant :
(a) à mettre en incubation séparément les cellules transfectées avec et capables d'exprimer la SEQUENCE I.D. N° 1 ou la SEQUENCE I.D. N° 5, et des cellules contenant un récepteur connu couplé à la protéine G en présence d'une entité chimique ;
(b) à ajouter aux milieux d'incubation de l'étape (a) un ligand ou agoniste naturel de la SEQUENCE I.D. N° 2 aux cellules transfectées de manière stable, et un ligand ou agoniste naturel aux cellules contenant un récepteur connu couplé à la protéine G ;
(c) à analyser l'effet de l'entité chimique sur les cellules ; et
(d) à déterminer si l'entité chimique inhibe ou bloque sélectivement le ligand ou agoniste naturel de la SEQUENCE I.D. N° 2.

20. Procédé suivant la revendication 19, dans lequel, dans l'étape (c), l'effet est analysé en mesurant le flux du calcium.

21. Méthode suivant la revendication 19, dans laquelle, dans l'étape (a), les cellules sont cotransfectées et les cellules contenant un récepteur connu couplé à la protéine G sont transfectées avec un vecteur codant pour la luciférase sous le contrôle d'un promoteur sensible à la protéine G ; et, dans l'étape (c), l'effet est analysé en mesurant l'activité de luciférase.

22. Méthode pour analyser un agent afin de déterminer l'aptitude de cet agent à jouer le rôle d'antagoniste d'un récepteur cellulaire comprenant un polypeptide suivant la revendication 13, méthode qui comprend les étapes consistant :
(a) à mettre séparément en incubation les cellules transfectées capables d'exprimer la SEQUENCE I.D. N° 1 ou la SEQUENCE I.D. N° 5 et des cellules témoins du même type mais transfectées avec et capables d'exprimer un récepteur témoin autre qu'un récepteur comprenant un polypeptide suivant la revendication 13 avec des quantités variables d'une entité chimique ;
(b) à ajouter aux cellules respectives de l'étape (a) un ligand marqué des récepteurs respectifs de ces cellules et
(c) à déterminer si l'entité chimique bloque ou inhibe la liaison du ligand marqué aux cellules transfectées exprimant la SEQUENCE I.D. N° 1 ou la SEQUENCE I.D. N° 5 mais non aux cellules témoins.

23. Séquence antisense à une séquence d'expression d'une séquence de nucléotides suivant la revendication 1 ou la revendication 6.
